# EUROPÄISCHE PATENTSCHRIFT

(11) **EP 1 103 540 B1**
(45) Veröffentlichungstag und Bekanntmachung des Hinweises auf die Patenterteilung: **01.06.2005**
(21) Anmeldenummer: 00122626.5
(22) Anmeldetag: 17.10.2000
(51) Int. Cl.: C07C 69/54, C08F 283/10, C08G 59/18

(54) **Vinylester mit hoher Vernetzungsdichte. Verfahren zu ihrer Herstellung und Verwendung**
Vinylesters having high cross-linking density, process for their preparation and their use
Esters vinyliques à haute densité de réticulation, procédé pour leur préparation ainsi que leur utilisation

(30) Priorität: 25.11.1999 DE 19956779
(43) Veröffentlichungstag der Anmeldung: 30.05.2001
(73) Patentinhaber: Bakelite AG, 58642 Iserlohn-Letmathe (DE)
(72) Erfinder: Grundke, Ulrich, 47137 Duisburg (DE); Liebetanz, Klaus-Peter, 47138 Duisburg (DE); Kalla, Volker, 47167 Duisburg (DE)

(56) Entgegenhaltungen:
- DE-A- 4 209 248
- DATABASE WPI Section Ch, Week 199650 Derwent Publications Ltd., London, GB; Class A21, AN 1996-502754 XP002159028 & JP 08 259663 A (DAINIPPON INK & CHEM INC), 8. Oktober 1996 (1996-10-08)
- DATABASE CHEMABS [Online] CHEMICAL ABSTRACTS SERVICE, COLUMBUS, OHIO, US; SHIMADA, HIROAKI ET AL: "Manufacture of water-thinned coating compositions for metals" retrieved from STN Database accession no. 113:117159 CA XP002159027 & JP 02 077417 A (DAINIPPON INK AND CHEMICALS, INC., JAPAN) 16. März 1990 (1990-03-16)
- DATABASE CHEMABS [Online] CHEMICAL ABSTRACTS SERVICE, COLUMBUS, OHIO, US; LEPLYANIN, G. V. ET AL: "Copolymers of (meth)acrylate diesters of.alpha.-glycols with vinyl monomers" retrieved from STN Database accession no. 87:202564 CA XP002159030 & SU 574 452 T (BASHKIR INSTITUTE OF CHEMISTRY, USSR) 30. September 1977 (1977-09-30)

## Beschreibung

Die Erfindung betrifft Vinylester, die durch Umsetzung von Epoxidverbindungen mit ungesättigten Verbindungen hergestellt werden, wobei die ungesättigten Verbindungen ungesättigte Carbonsäuren und/oder Carbonsäureanhydride sind.

Derartige Verbindungen werden als photohärtende Präpolymere oder als Lackrohstoffe eingesetzt. Sie zeichnen sich durch gute elektrische Eigenschaften sowie durch hohe Haftung auf nahezu allen Substraten und durch gute Chemikalienbeständigkeit aus. Dennoch besteht der Bedarf an hochreaktiven Produkten, die das gleiche gute Eigenschaftsspektrum haben, aber hinsichtlich ihrer Chemikalien- und Temperaturbeständigkeit noch verbessert sind. Die Aufgabe der Erfindung besteht daher darin, derartige Verbindungen bereitzustellen.

Die Lösung der Aufgabe erfolgt durch Vinylester gemäß der Ansprüche 1 bis 5 und durch ein Verfahren zu ihrer Herstellung gemäß der Ansprüche 7 bis 11. Die erfindungsgemäßen Vinylester werden bevorzugt eingesetzt als Lackrohstoffe, als photohärtende Komponente in Beschichtungsmitteln sowie als Bindemittelkomponente in chemischen Reaktionsharzmassen, insbesondere in solchen, die zur Herstellung von chemischen Verankerungsmitteln dienen, wie dies in den Ansprüchen 12 bis 15 beansprucht ist.

Umsetzungsprodukte aus Epoxidverbindungen mit ungesättigten Säureanhydriden und/oder Carbonsäuren sind an sich bekannt. Jedoch sind bei diesen Produkten die Epoxidgruppen nur partiell verestert wobei die Epoxidgruppen durch β-Hydroxy-Vinylester-Gruppen substituiert sind.

So offenbart JP-A 02077417 Beschichtungsmittel für Metall-Container, die ein Bindemittel enthalten, das durch Umsetzung von 90 - 99,95 Gew.% eines aromatischen Epoxidharzes mit 0,05 - 10 Gew.% Acrylsäure- und/oder Methacrylsäure-Anhydrid erhalten wird.

DE-A 42 09 248 beschreibt Pfropfpolymere, bei denen in einem ersten Schritt Epoxidharze mit geringen Mengen Acrylsäure- und/oder Methacrylsäure-Anhydrid erhitzt werden und diese Produkte dann mit anderen ethylenisch ungesättigten Verbindungen umgesetzt werden.

Aus JP-A 08259663 sind strahlenhärtbare Vinylester bekannt, bei denen in einem ersten Reaktionsschritt Epoxidharze mit 0,1 - 0,8 mol Acrylsäure- und/oder Methacrylsäure-Anhydrid und mit 0,2 - 0,9 mol ungesättigter Carbonsäure pro mol Epoxidgruppe umgesetzt werden. Danach erfolgt eine Umsetzung dieser Reaktionsprodukte bzw. der β-Hydroxygruppen dieser Vinylester mit mehrwertigen Säureanhydriden.

Es wurde gefunden, daß man im wesentlichen β-hydroxygruppenfreie Vinylester erhält, wenn man Epoxidverbindungen mit zu den Epoxidgruppen stöchiometrischen Mengen an ethylenisch ungesättigten Carbonsäureanhydriden umsetzt.
So erhält man z.B. bei der Umsetzung von Bisphenol-A- oder Bisphenol-F-Diglycidylethern mit Acrylsäure oder Methacrylsäure in zu den Epoxidgruppen stöchiometrischen Mengen Vinylester mit einem Äquivalentgewicht von 250 - 300 g Harz/Doppelbindung. Bei der entsprechenden Umsetzung mit Acrylsäureanhydrid oder Methacrylsäureanhydrid entstehen Vinylester mit einem Äquivalentgewicht von 170 - 200 g Harz/Doppelbindung.
Bei den erhaltenen Produkten sind im wesentlichen die Epoxidgruppen durch zwei Vinylestergruppen substituiert. Diese Vinylester zeigen das gleich gute Eigenschaftsprofil wie die bekannten Vinylester, ergeben aber bei der Härtung eine erhöhte Vernetzungsdichte. Daraus resultiert eine höhere Chemikalien- und Temperaturbeständigkeit

Überraschenderweise haben die erfindungsgemäßen Vinylester mit hoher Vernetzungsdichte eine deutlich niedrigere Viskosität als einfache, β-Hydroxylgruppen enthaltende Vinylester.
Dadurch erhält man z.B. in einer Lackformulierung bei gleichem Monomeranteil eine niedrigere Verarbeitungsviskosität. D.h., zum Erreichen gleicher Endviskosität kann man den Anteil an Monomeren oder Lösemitteln verringern. Dies führt zu einer nochmaligen Erhöhung der Chemikalien- und Temperaturbeständigkeit.

Es ist ein weiterer überraschender Vorteil, daß die erfindungsgemäßen Vinylester mit hoher Vernetzungsdichte trotzdem eine gegenüber den bekannten Vinylestern verbesserte Lagerbeständigkeit aufweisen. Sie können bei Raumtemperatur bis zu 52 Wochen gelagert werden, ohne daß ihre Viskosität wesentlich ansteigt.

Als Epoxidverbindung zur Herstellung der erfindungsgemäßen Vinylester können alle an sich bekannten monomeren und oligomeren Verbindungen, Glycidylether, Glycidylester oder N-glycidyl-Verbindungen mit einer oder mehreren Epoxidgruppen eingesetzt werden. Bevorzugt werden Epoxidverbindungen mit zwei und mehr Epoxidgruppen im Molekül, insbesondere Diglycidylether von Bisphenolen sowie Glycidylether von Novolaken auf Basis von Phenol, Kresol oder Bisphenolen.

Als ethylenisch ungesättigte Carbonsäureanhydride können im Prinzip alle Carbonsäureanhydride von Monocarbonsäuren eingesetzt werden, die eine oder mehrere ethylenisch ungesättigte Gruppe enthalten. Die bevorzugten Anhydride sind Acrylsäureund Methacrylsäure-Anhydrid.

Die Umsetzung der Epoxidverbindungen mit den ethylenisch ungesättigten Säureanhydriden erfolgt in an sich bekannter Weise durch Zusammenfügen der Komponenten in entsprechender Menge, gegebenenfalls unter Zuhilfenahme eines Lösemittels, eines an sich bekannten Katalysators und eines als Radikalfänger wirkenden Stabilisierungsmittels wie z.B. Hydrochinon, p-Benzochinon, Methylhydrochinon, Brenzkatechin und dergleichen.
Der Katalysator wird ausgewählt aus den an sich bekannten Gruppen Metallsalze, wie z.B. Dibutylzinndilaurat, Zinnoktoat, Phosphorverbindungen wie z.B. Triphenylphosphin oder Ethyltriphenylphosphoniumbromid, tert. Aminen, wie z.B. Dimethylaminopyridin oder Benzyldimethylamin oder quarternäre Ammoniumverbindungen, wie z.B. Tetraethylammoniumbromid.
Danach erfolgt ein mehrstündiges Erwärmen des jeweiligen Reaktionsgemisches bei Temperaturen im Bereich von 80 - 120°C. Bevorzugt wird bei dieser Reaktion Sauerstoff oder ein Sauerstoff enthaltendes Gas durch das Reaktionsgemisch geleitet, um eine Polymerisation zu verhindern.

Bei der Umsetzung von hydroxylgruppenfreien Epoxidverbindungen mit den ethylenisch ungesättigten Säureanhydriden wird bevorzugt eine geringe Menge einer Carbonsäure zugegeben, um den Start der Veresterungsreaktion zu beschleunigen. Bevorzugt sind hier ethylenisch ungesättigte Carbonsäuren, insbesondere Acryl- und/oder Methacrylsäure. Mit steigender Menge an zugegebener Carbonsäure wird zwar einerseits der Start der Veresterungsreaktion beschleunigt, andererseits aber der Gehalt an Vinylestergruppen vermindert. Unter Abwägung dieser beiden Kriterien wird daher die Menge der zugegebenen Carbonsäure so gewählt, daß sie im Bereich von bis zu 0,1 mol, bevorzugt bis zu 0,05 mol, insbesondere bevorzugt bis zu 0,01 mol pro mol der eingesetzten Epoxidgruppen liegt. Die Mengen der eingesetzten ethylenisch ungesättigten Säureanhydride muß dann entsprechend reduziert werden, so daß pro mol Epoxidgruppe 0,9 bis 1 mol ethylenisch ungesättigtes Säureanhydrid und 0,1 bis 0 mol Carbonsäure, bevorzugt 0,95 bis 1 mol ethylenisch ungesättigtes Säureanhydrid und 0,05 bis 0 mol Carbonsäure und insbesondere bevorzugt 0,99 bis 1 mol ethylenisch ungesättigtes Säureanhydrid und 0,01 bis 0 mol Carbonsäure eingesetzt werden.
Enthalten die eingesetzten Epoxidverbindungen Hydroxylgruppen, wie dies z.B. bei Epoxidharzen der Fall ist, die durch Umsetzung von Bisphenolen mit Epichlorhydrin und nachfolgender Advancement-Reaktion hergestellt sind, so wird bevorzugt auf den Einsatz einer Startersäure verzichtet.

Die Aufarbeitung des Reaktionsgemisches erfolgt ebenfalls in an sich bekannter Weise durch schonendes Abdestillieren des eventuell verwendeten Lösemittels und gegebenenfalls durch Zugabe eines weiteren Stabilisierungsmittels.

In der Regel werden flüssige Produkte erhalten, die durch Bestrahlung , Wärme oder mittels Katalysator härtbar sind. Aufgrund ihrer Eigenschaften werden sie bevorzugt eingesetzt als Lackrohstoffe, als photohärtende Komponente in Beschichtungsmitteln sowie als Bindemittel oder Bindemittelkomponente in chemischen Reaktionsharzmassen, insbesondere solchen zur Herstellung von chemischen Verankerungsmitteln.

### Beispiele

### Beispiel 1 (Vergleichsbeispiel)

In einen mit Rührer, Thermometer, Gaseinleitungsrohr und Zugabevorrichtung versehenen Reaktor werden 368 g (1 mol) Bisphenol-A-diglycidylether, 4,0 g Triphenylphosphin und 0,5 g Hydrochinonmonomethylether eingewogen und auf etwa 90°C erwärmt. Unter Rühren und ständiger Lufteinleitung werden innerhalb von 2 h 172 g (2 mol) Methacrylsäure zugegeben.
Das Reaktionsgemisch wird unter Rühren und Lufteinleitung weiter bei 90°C gehalten bis es eine Säurezahl < 5 mg KOH/g hat.
Es entsteht ein Vinylesterharz mit einer Viskosität bei 40°C von etwa 47.000 mPas und einer Doppelbindungsdichte von 270 g Harz pro Doppelbindung.
Bei einer Lagerung bei 100°C wird folgender Viskositätsanstieg gemessen:

| | |
|---|---|
| 7 d 100°C | 154% |
| 14 d 100°C | 200% |
| 28 d 100°C | geliert. |

### Beispiel 2

In einen mit Rührer, Thermometer, Gaseinleitungsrohr und Zugabevorrichtung versehenen Reaktor werden 368 g (1 mol) Bisphenol-A-diglycidylether, 4,0 g Triphenylphosphin und 0,5 g Hydrochinonmonomethylether eingewogen und auf etwa 90°C erwärmt. Unter Rühren und ständiger Lufteinleitung werden innerhalb von 15 min 17,2 g (0,2 mol) Methacrylsäure zugegeben. Sofort anschließend werden unter gleichen Bedingungen innerhalb von 2 weiteren h 277,2 g (1,8 mol) Methacrylsäureanhydrid zugegeben. Das Reaktionsgemisch wird unter Rühren und Lufteinleitung weiter bei 90°C gehalten bis es eine Säurezahl < 5 mg KOH/g hat.
Es entsteht ein Vinylesterharz mit einer Viskosität bei 40°C von 9.000 mPas und einer Doppelbindungsdichte von 175 g Harz pro Doppelbindung.
Bei einer Lagerung bei 100°C wird folgender Viskositätsanstieg gemessen:

| | |
|---|---|
| 7 d 100°C | 118% |
| 14 d 100°C | 138% |
| 28 d 100°C | 149%. |

### Beispiel 3

Die beiden Vinylester aus den Beispielen 1 und 2 werden mit Trimethylolpropantrimethacrylat (TRIM) verdünnt:
a. 50 GT Vinylester aus Beispiel 1
   50 GT TRIM
   Viskosität der Mischung bei 25°C: 2.100 mPas
b. 50 GT Vinylester aus Beispiel 2
   50 GT TRIM
   Viskosität der Mischung bei 25°C: 950 mPas
c. 60 GT Vinylester aus Beispiel 2
   40 GT TRIM
   Viskosität der Mischung bei 25°C: 2.000 mPas.

Zur Überprüfung der Chemikalienbeständigkeit werden die Mischungen a - c mit Co-Aminbeschleuniger und tert.-Butylperoxibenzoat auf eine Gelierzeit von 30 min eingestellt. Mit den so hergestellten Mischungen a', b' und c' werden Probekörper mit den Abmessungen 50x50x3 mm hergestellt.
Nach Aushärtung ( 7 d bei Raumtemperatur) werden die Probekörper aus a', b' und c' jeweils 50 und 100 h bei 100°C in 10%iger wäßriger Natronlauge gelagert, danach abgespült, mit Zellstoffpapier abgetrocknet und anschließend gewogen.
Es werden folgende Gewichtszunahmen ermittelt:

| Gewichtszunahme [ %] | Probekörper aus Mischung | | |
|---|---|---|---|
| | a' | b' | c' |
| nach 50 h | 2,6 | 1,2 | 1,05 |
| nach 100 h | 3,2 | 1,4 | 1,1. |

## Patentansprüche

1. Vinylester, hergestellt durch Umsetzung von Epoxidverbindungen mit Carbonsäuren und ethylenisch ungesättigten Gruppen enthaltenden Carbonsäureanhydriden, **dadurch gekennzeichnet, dass** die Umsetzung mit kleiner/gleich 0,1 mol (pro Mol der eingesetzten Epoxidgruppen) Carbonsäure und mit größer/gleich 0,9 mol und kleiner 1 mol (pro Mol der eingesetzten Epoxidgruppen) ethylenisch ungesättigte Gruppen enthaltenden Carbonsäureanhydriden erfolgt, bei denen im wesentlichen die Epoxidgruppen durch zwei Vinylestergruppen substituiert sind.

2. Vinylester nach Anspruch 1, hergestellt durch Umsetzung von Epoxidverbindungen mit 0,95 bis kleiner 1 mol (pro Mol der eingesetzten Epoxidgruppen) ungesättigtem Säureanhydrid und kleiner/gleich 0,05 mol (pro mol der eingesetzten Epoxidgruppen) Carbonsäure.

3. Vinylester nach Anspruch 1, hergestellt durch Umsetzung von Epoxidverbindungen mit 0,99 bis kleiner 1 mol (pro mol der eingesetzten Epoxidgruppen) ungesättigtem Säureanhydrid und kleiner/gleich 0,01 mol (pro mol der eingesetzten Epoxidgruppen) Carbonsäure.

4. Vinylester nach einem der Ansprüche 1 bis 3, **dadurch gekennzeichnet, dass** das Säureanhydrid Acrylsäureund/oder Methacrylsäureanhydrid ist.

5. Vinylester nach einem der Ansprüche 1 bis 3, **dadurch gekennzeichnet, dass** die Carbonsäure Acryl- und/oder Methacrylsäure ist.

6. Verfahren zur Herstellung der Vinylester gemäß Anspruch 1 durch Umsetzung von Epoxidverbindungen mit ethylenisch ungesättigten Säureanhydriden und Carbonsäuren, **dadurch gekennzeichnet, dass** pro mol Epoxidgruppe 0,9 bis kleiner 1 mol ungesättigtes Säureanhydrid und kleiner/gleich 0,1 mol Carbonsäure eingesetzt werden.

7. Verfahren-nach Anspruch 6, **dadurch gekennzeichnet**, das pro mol Epoxidgruppe 0,95 bis kleiner 1 mol ungesättigtes Säureanhydrid und kleiner/gleich 0,05 mol Carbonsäure eingesetzt werden.

8. Verfahren nach Anspruch 7, **dadurch gekennzeichnet, daß** pro mol Epoxidgruppe 0,99 bis kleiner 1 mol ungesättigtes Säureanhydrid und kleiner/gleich 0,01 mol Carbonsäure eingesetzt werden.

9. Verfahren nach einem der Ansprüche 6 bis 8, **dadurch gekennzeichnet, dass** als Säureanhydrid Acrylsäure- und/oder Methacrylsäureanhydrid eingesetzt werden.

10. Verfahren nach einem der Ansprüche 6 bis 9, **dadurch gekennzeichnet, dass** als Carbonsäure Acryl- und/oder Methacrylsäure eingesetzt werden.

11. Verwendung der Vinylester gemäß der Ansprüche 1 bis 5 als Lackrohstoffe.

12. Verwendung der Vinylester gemäß der Ansprüche 1 bis 5 als photohärtende Komponente in Beschichtungsmitteln.

13. Verwendung der Vinylester gemäß der Ansprüche 1 bis 5 als Bindemittel oder Bindemittelkomponente in chemischen Reaktionsharzmassen.

14. Verwendung der Vinylester gemäß der Ansprüche 1 bis 5 als Bindemittel oder Bindemittelkomponente in Reaktionsharzmassen zur Herstellung von chemischen Verankerungsmitteln.

## Claims

1. Vinyl esters, prepared by reacting epoxide compounds with carboxylic acids and with carboxylic anhydrides containing ethylenically unsaturated groups, **characterized in that** the reaction takes place with less than/equal to 0.1 mol (per mole of epoxide groups employed) of carboxylic acid and with greater than/equal to 0.9 mol and less than 1 mol (per mole of epoxide groups employed) of carboxylic anhydrides containing ethylenically unsaturated groups, in which essentially the epoxide groups are substituted by two vinyl ester groups.

2. Vinyl esters according to Claim 1, prepared by reacting epoxide compounds with 0.95 to less than 1 mol (per mole of epoxide groups employed) of unsaturated acid anhydride and less than/equal to 0.05 mol (per mole of epoxide groups employed) of carboxylic acid.

3. Vinyl esters according to Claim 1, prepared by reacting epoxide compounds with 0.99 to less than 1 mol (per mole of epoxide groups employed) of unsaturated acid anhydride and less than/equal to 0.01 mol (per mole of epoxide groups employed) of carboxylic acid.

4. Vinyl esters according to any one of Claims 1 to 3, **characterized in that** the acid anhydride is acrylic anhydride and/or methacrylic anhydride.

5. Vinyl esters according to any one of Claims 1 to 3, **characterized in that** the carboxylic acid is acrylic acid and/or methacrylic acid.

6. Process for preparing the vinyl esters according to Claim 1 by reacting epoxide compounds with ethylenically unsaturated acid anhydrides and carboxylic acids, **characterized in that** per mole of epoxide group 0.9 to less than 1 mol of unsaturated acid anhydride and less than/equal to 0.1 mol of carboxylic acid are used.

7. Process according to Claim 6, **characterized in that** per mole of epoxide group 0.95 to less than 1 mol of unsaturated acid anhydride and less than/equal to 0.05 mol of carboxylic acid are employed.

8. Process according to Claim 7, **characterized in that** per mole of epoxide group 0.99 to less than 1 mol of unsaturated acid anhydride and less than/equal to 0.01 mol of carboxylic acid are employed.

9. Process according to any one of Claims 6 to 8, **characterized in that** acrylic anhydride and/or methacrylic anhydride are used as acid anhydride.

10. Process according to any one of Claims 6 to 9, **characterized in that** acrylic acid and/or methacrylic acid are used as carboxylic acid.

11. Use of the vinyl esters according to Claims 1 to 5 as coatings raw materials.

12. Use of the vinyl esters according to Claims 1 to 5 as photocuring component in coating materials.

13. Use of the vinyl esters according to Claims 1 to 5 as binders or a binder component in chemical reactive resin compositions.

14. Use of the vinyl esters according to Claims 1 to 5 as binders or a binder component in reactive resin compositions for producing chemical anchoring materials.

## Revendications

1. Esters vinyliques, préparés par réaction de composés de type époxyde avec des acides carboxyliques et des anhydrides d'acides carboxyliques contenant des groupes à insaturation éthylénique, **caractérisés en ce que** la réaction s'effectue avec ≤ 0,1 mole (par mole des groupes époxy utilisés) d'acide carboxylique et avec ≥ 0,9 mole et < 1 mole (par mole des groupes époxy utilisés) d'anhydrides d'acides carboxyliques contenant des groupes à insaturation éthylénique, dans lesquels essentiellement les groupes époxy sont substitués par deux groupes ester vinylique.

2. Esters vinyliques selon la revendication 1, préparés par réaction de composés de type époxyde avec 0,95 à < 1 mole (par mole des groupes époxy utilisés) d'anhydride d'acide insaturé et ≤ 0,05 mole (par mole des groupes époxy utilisés) d'acide carboxylique.

3. Esters vinyliques selon la revendication 1, préparés par réaction de composés de type époxyde avec 0,99 à < 1 mole (par mole des groupes époxy utilisés) d'anhydride d'acide insaturé et ≤ 0,01 mole (par mole des groupes époxy utilisés) d'acide carboxylique.

4. Esters vinyliques selon l'une quelconque des revendications 1 à 3, **caractérisés en ce que** l'anhydride d'acide est l'anhydride d'acide acrylique et/du l'anhydride d'acide méthacrylique.

5. Esters vinyliques selon l'une quelconque des revendications 1 à 3, **caractérisés en ce que** l'acide carboxylique est l'acide acrylique et/ou l'acide méthacrylique.

6. Procédé pour la préparation d'esters vinyliques selon la revendication 1, par réaction de composés de type époxyde avec des acides carboxyliques et des anhydrides d'acides à insaturation éthylénique, **caractérisé en ce que**, par mole de groupe époxy, on utilise de 0,9 à < 1 mole d'anhydride d'acide insaturé et ≤ 0,1 mole d'acide carboxylique.

7. Procédé selon la revendication 6, **caractérisé en ce qu'**on utilise, par mole de groupe époxy, 0,95 à < 1 mole d'anhydride d'acide insaturé et ≤ 0,05 mole d'acide carboxylique.

8. Procédé selon la revendication 7, **caractérisé en ce que**, par mole de groupe époxy, on utilise 0,99 à < 1 mole d'anhydride d'acide insaturé et ≤ 0,01 mole d'acide carboxylique.

9. Procédé selon l'une quelconque des revendications 6 à 8, **caractérisé en ce qu'**on utilise en tant qu'anhydride d'acide l'anhydride d'acide acrylique et/ou l'anhydride d'acide méthacrylique.

10. Procédé selon l'une quelconque des revendications 6 à 9, **caractérisé en ce qu'**on utilise en tant qu'acide carboxylique l'acide acrylique et/ou l'acide méthacrylique.

11. Utilisation des esters vinyliques selon les revendications 1 à 5, en tant que matières premières pour peintures.

12. Utilisation des esters vinyliques selon les revendications 1 à 5, en tant que composant photodurcisseur dans des compositions de revêtement.

13. Utilisation des esters vinyliques selon les revendications 1 à 5, en tant que liant ou composant de liant dans des matières de type résine pour réaction chimique.

14. Utilisation des esters vinyliques selon les revendications 1 à 5, en tant que liant ou composant de liant dans des matières de type résine réactive pour la production d'agents chimiques d'accrochage.
